(19) Europäisches Patentamt
European Patent Office
Office européen des brevets

(11) **EP 2 940 454 A1**

(12) **EUROPEAN PATENT APPLICATION**
published in accordance with Art. 153(4) EPC

(43) Date of publication:
**04.11.2015 Bulletin 2015/45**

(51) Int Cl.:
*G01N 21/359* (2014.01)  *G01N 21/3554* (2014.01)

(21) Application number: **13866640.9**

(86) International application number:
**PCT/JP2013/006955**

(22) Date of filing: **27.11.2013**

(87) International publication number:
**WO 2014/103165 (03.07.2014 Gazette 2014/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.12.2012 JP 2012286265**

(71) Applicant: **Panasonic Intellectual Property Management Co., Ltd.**
**Osaka-shi, Osaka 540-6207 (JP)**

(72) Inventors:
• **OCHI, Kazuhiro**
  **7F OBP Panasonic Tower, 1-61, Shiromi 2-chome, Chuo-ku, Osaka 540-6207 (JP)**
• **TAKAHASHI, Tatsuya**
  **7F OBP Panasonic Tower, 1-61, Shiromi 2-chome, Chuo-ku, Osaka 540-6207 (JP)**

(74) Representative: **Grünecker Patent- und Rechtsanwälte**
**PartG mbB**
**Leopoldstraße 4**
**80802 München (DE)**

(54) **CALORIE CALCULATION DEVICE**

(57)     A calorie calculation device (1) having a measurement unit (20), a weight detection unit (30), and a control unit (70). The measurement unit (20) measures the water content of an analysis target (S). The weight detection unit (30) measures the weight of the analysis target (S). The control unit (70) calculates the calories of the analysis target (S) by using the measurement results of both the measurement unit and the weight detection unit.

Fig.1

# Description

The present invention relates to a calorie calculation device.

Patent document 1 discloses one example of a component analyzer that measures components of an analyzed subject. The human body uses the components of an analyzed subject as an energy source. In the prior art, a known method measures protein, carbohydrate, fat, and water included in an analyzed subject with a component analyzer and calculates the calories of the analyzed subject based on the measurement result.

Patent Document 1: Japanese Laid-Open Patent Publication No. 2002-122538

The calorie calculation method described above measures each of protein, carbohydrate, fat, and water. Thus, a complicated configuration is used to calculate the calories.

It is an object of the present invention to provide a calorie calculation device that allows calories to be calculated with a simple configuration.

One aspect of the present invention is a calorie calculation device. The calorie calculation device includes a measurement unit that performs a measurement on, at most, three components among water, protein, carbohydrate, and fat that are included in an analyzed subject; either a weight detector that measures weight of the analyzed subject or a volume detector that measures volume of the analyzed subject; and a calorie calculator that calculates calories of the analyzed subject using a measurement result of the measurement unit and either a measurement result of the weight detector or a measurement result of the volume detector.

In the above configuration, preferably, the measurement unit performs a measurement on at least water among water, protein, carbohydrate, and fat that are included in the analyzed subject.

In the above configuration, preferably, the measurement unit performs a measurement on one or two of protein, carbohydrate, and fat that are included in the analyzed subject.

In the above configuration, preferably, the measurement unit performs a measurement on water that is included in the analyzed subject and fat that is included in the analyzed subject.

In the above configuration, preferably, the measurement unit measures the analyzed subject based on spectrum data obtained with near-infrared light.

In the above configuration, preferably, the measurement unit performs a measurement on the analyzed subject based on spectrum data obtained with near-infrared light from 700 to 1100 nm.

In the above configuration, preferably, the calorie calculation device includes either a food type information input unit that receives food type information of the analyzed subject or a food type information detector that detects food type information of the analyzed subject. The calorie calculator uses the food type information of the analyzed subject to correct the calories of the analyzed subject.

In the above configuration, preferably, the measurement unit measures a ratio of water included in an analyzed subject, and the calorie calculator uses the ratio of water included in the analyzed subject and either a measurement result of the weight detector or a measurement result of the volume detector to calculate the calories of the analyzed subject.

In the above configuration, preferably, the measurement unit measures a ratio of water included in an analyzed subject and measures a ratio of fat included in the analyzed subject. The calorie calculator uses the ratio of water included in the analyzed subject, the ratio of fat included in the analyzed subject, and either a measurement result of the weight detector or a measurement result of the volume detector to calculate the calories of the analyzed subject.

The calorie calculation device allows calories to be calculated with a simple configuration.

Fig. 1 is a schematic diagram entirely showing a first embodiment of a calorie calculation device.
Fig. 2 is a perspective view entirely showing the structure of a sample tray in the first embodiment.
Fig. 3 is a graph showing the relationship of the water ratio of foods and the calories of the foods.
Fig. 4 is a schematic diagram entirely showing a further embodiment of a calorie calculation device.
Fig. 5 is a schematic diagram entirely showing another embodiment of a calorie calculation device.

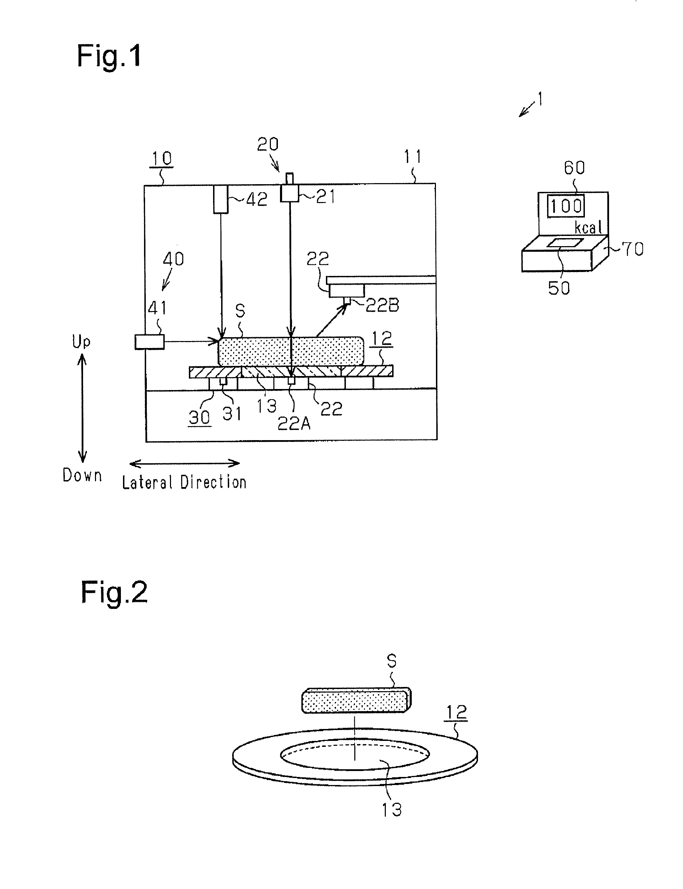

The configuration of a calorie calculation device 1 will now be described with reference to Figs. 1 and 2. The calorie calculation device 1 calculates the calories of a food, which serves as an analyzed subject S. The food may be a solid or a liquid.

As shown in Fig. 1, the calorie calculation device 1 includes a main body 10, a measurement unit 20, a weight detector 30, a position sensor 40, a control unit 70, an operation unit 50, and a display 60. The control unit 70 corresponds to a "calorie calculator".

The main body 10 includes a case 11 and a sample tray 12.

The sample tray 12 and the analyzed subject S are located in the case 11. The case 11 includes a door (not shown). The case 11 includes a door (not shown). When the door is closed, the interior of the case 11 is shielded from light.

As shown in Fig. 2, the sample tray 12 includes a transparent portion 13. The sample tray 12 is, for example, disk-shaped. The transparent portion 13 is located in the central portion of the sample tray 12, which is disk-shaped. The transparent portion 13 is formed from a material that transmits near-infrared light. The material of the transparent portion 13 may be, for example, silica glass that absorbs light in the infrared band, the amount of which is relatively small.

As shown in Fig. 1, the measurement unit 20 includes a light emitter 21 and light receivers 22. The light emitter 21 is located above the sample tray 12. The light emitter 21 irradiates the analyzed subject S, which is placed on an upper surface of the sample tray 12, with light. The light emitted from the light emitter 21 has a wavelength included in at least a portion of the range from 700 to 1100 nm, which is near-infrared light. The light source of the light emitter 21 may be, for example, a halogen lamp.

The light receivers 22 include a first light reception element 22A and a second light reception element 22B.

The first light reception element 22A is located below the sample tray 12. In the light emitted from the light emitter 21, the first light reception element 22A receives the light transmitted through the analyzed subject S. The first light reception element 22A transmits a signal that corresponds to the received light to the control unit 70. For example, a silicon element is used as the first light reception element 22A.

The second light reception element 22B is located above the sample tray 12. In the light emitted from the light emitter 21, the second light reception element 22B receives the light reflected and diffused by the analyzed subject S. The second light reception element 22A transmits a signal that corresponds to the received light to the control unit 70. For example, a silicon element is used as the second light reception element 22B.

The weight detector 30 includes a piezoelectric element 31. The sample tray 12 is arranged on an upper surface of the weight detector 30. Thus, pressure corresponding to the weight of the sample tray 12 and the analyzed subject S placed on the sample tray 12 is applied to the weight detector 30. The piezoelectric element 31 provides the control unit 70 with a signal corresponding to the pressure resulting from the weight of the sample tray 12 and the weight of the analyzed subject S placed on the sample tray 12.

The position sensor 40 includes a first position sensor 41 and a second position sensor 42.

The first position sensor 41 is coupled to an inner surface of the case 11. The first position sensor 41 is located on an inner side surface of the case 11 opposed to the peripheral surface of the sample tray 12. The first position sensor 41 provides the control unit 70 with a signal corresponding to the distance from the analyzed subject S.

The second position sensor 42 is coupled to an inner surface of the case 11. The second position sensor 42 is located above the sample tray 12. The second position sensor 42 provides the control unit 70 with a signal corresponding to the distance from the analyzed subject S.

The operation unit 50 includes a measurement start button (not shown). When a user pushes the measurement start button, the operation unit 50 provides the control unit 70 with a signal indicating the start of a measurement.

The display 60 includes a liquid crystal screen. The display 60 shows the calculation result of the calories of the analyzed subject S on the liquid crystal screen.

The control unit 70 is connected by cables (not shown) to the measurement unit 20, the weight detector 30, the position sensor 40, the operation unit 50, and the display 60. The control unit 70 calculates the calories of the analyzed subject S based on signals from the light reception elements 22A and 22B. The control unit 70 calculates the lateral position of the analyzed subject S based on a signal from the first position sensor 41. The control unit 70 calculates the weight of the analyzed subject S based on a signal from the piezoelectric element 31 of the weight detector 30. The control unit 70 calculates the position of the analyzed subject S in the lateral direction of the case 11 based on the signal from the first position sensor 41. The control unit 70 calculates the position of the analyzed subject S in the vertical direction of the case 11 based on the signal from the second position sensor 42.

The procedures for measuring the calories of the analyzed subject S with the control unit 70 will now be described. When the operation unit 50 is operated and a measurement start signal is received, the control unit 70 calculates the calories of the analyzed subject S in the order of procedures 1 to 5.

Procedure 1: The control unit 70 calculates the weight of the analyzed subject S based on a signal from the piezoelectric element 31 and the preset weight of the sample tray 12.

Procedure 2: The control unit 70 detects the position of the analyzed subject S based on a signal from the position sensor 40. More specifically, the control unit 70 calculates the distance from the analyzed subject S to the light emitter 21, the distance from the analyzed subject S to the light reception element 22A, and the distance from the analyzed subject S to the light reception element 22B.

Procedure 3: The control unit 70 emits near-infrared light from the light emitter 21 of the measurement unit 20. Further, the control unit 70 detects the signal from the light receiver 22 that is based on the received light amount.

Procedure 4: The control unit 70 calculates the calories of the analyzed subject S based on the weight of the analyzed subject S, the position of the analyzed subject S, and the light reception amount from the light receiver 22.

Procedure 5: The control unit 70 shows the calculated calories on the display 60.

A method for calculating calories will now be described.

The prior art method for calculating the calories of a food measures the weight of each component and coverts the weight of each component to a calorie conversion coefficient in order to calculate the total calories. The components include protein, carbohydrate, fat, and water.

A configuration that uses, for example, the weight of each component with near-infrared light measures the content ratio of each component based on the overlapped absorption spectrum of characteristic wavelengths of protein, carbohydrate, fat, and water. The weight of each component is obtained from the content ratio of each component and the total weight. The components overlap one another in the absorption spectrums. Thus, a highly accurate sensor needs to be used to separate components in the absorption spectrum. Further, without a high accurate sensor, complicated algorithms become necessary. This lengthens the analysis time. In a configuration that measures the content ratio of each component, a light emitter and a light receiver uses a filter corresponding to each component for the component measurement. This enlarges the measurement device. Additionally, there is a need to measure all of the components. Thus, it takes time to measure the calories of a food.

When compared to the band of 1100 nm or higher, in the near-infrared band, particularly, in the band of 700 to 1100 nm, which is referred to as the shortwave band, near-infrared light is easily transmitted through the analyzed subject S. In the band of 700 to 1100 nm, protein does not have a clear peak in the absorption spectrum. Thus, when using the near-infrared light of 700 to 1100 nm, the calculation of the content ratio of protein is difficult.

The inventors of the present application have found that the weight calorie of each food calculated by the prior art method is correlated with the water ratio of the analyzed subject S.

More specifically, food typically includes water, protein, carbohydrate, and fat. When a predetermined weight of water is decomposed, water has a calorie production amount (hereinafter referred to as "the calorie contribution rate"). Water takes up about one-half of the weight in many foods. Thus, the water content of a food is correlated with the calories of the entire food.

Carbohydrate has a lower calorie contribution rate than fat. Foods such as rice and noodles, which usually include water, include a large amount of carbohydrate. Thus, foods such as rice and noodles have a relatively high water ratio. Fat has a relatively high calorie contribution ratio. Thus, foods including a large amount of fat have a relatively small water ratio. Accordingly, in food having a relatively low water ratio and food having a relatively high water ratio, the water ratio is highly correlated with the calories of the food. That is, the water ratio indicates a high correlation with the calories of the entire food.

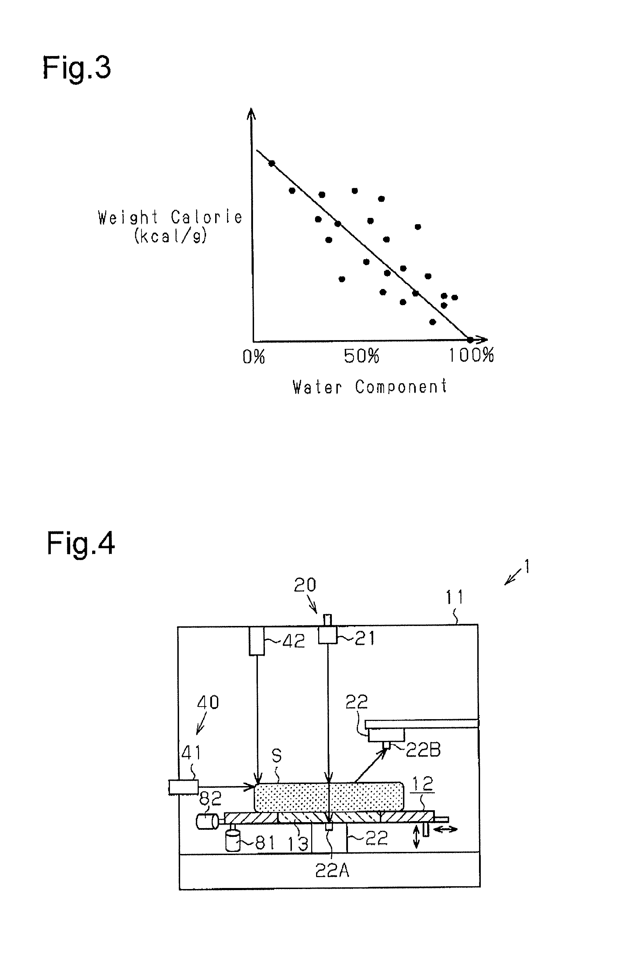

Fig. 3 shows the relationship of the water ratio and the weight calorie in a number of foods. The water ratio and weight calorie in Fig. 3 are measured through known methods. The water ratio indicates the ratio of the weight of water relative to the weight of the entire food. The water ratio indicates the calories per weight of the food.

As shown in Fig. 3, the water ratio is correlated with the weight calorie in a number of foods. More specifically, as the water ratio of a food increases, the weight calorie of the food decreases.

The control unit 70 spectrally measures near-infrared light based on a signal from the light receiver 22 and generates spectrum data for wavelengths from 700 to 1100 nm. The control unit 70 uses the spectrum data and the position of the analyzed subject S to calculate the water ratio of the analyzed subject S. More specifically, the absorbance of the received light changes in accordance with the position of the analyzed subject S. Thus, the control unit 70 corrects the spectrum data based on the position of the analyzed subject S and calculates the water ratio based on the absorbance of the wavelength suitable for the water calculation. More specifically, as the distance from the light emitter 21 to the analyzed subject S increases, the amount of light received by the two light reception elements 22A and 22B decreases. Further, as the distance from the analyzed subject S to the light reception elements 22A and 22B increases, the amount of light received by the light reception elements 22A and 22B decreases. Thus, for example, when the distance between the analyzed subject S and the light emitter 21 and between the analyzed subject S and the light reception elements 22A and 22B increases, the absorbance is corrected to be larger than when the distance is small between the analyzed subject S and the light emitter 21 and between the analyzed subject S and the light reception elements 22A and 22B.

Then, the control unit 70 calculates the weight calorie of the analyzed subject S using a relational equation of an approximate line obtained from the relationship of the water ratio and the weight calorie in the foods shown in Fig. 3 and the water ratio of the analyzed subject S calculated by the calorie calculation device 1. The control unit 70 then calculates the calories of the analyzed subject S using the weight calorie of the analyzed subject S and the weight of the analyzed subject S.

The calorie calculation device 1 has the advantages described below.

(1) The calorie calculation device 1 calculates the calories of the analyzed subject S based on the water ratio of the analyzed subject S. This allows calories to be calculated with a simpler configuration than a configuration in which the protein, carbohydrate, fat, and water in the analyzed subject S are all measured to calculate the calories.

(2) A known calorie calculation method of the analyzed subject S crushes the analyzed subject S and analyzes the components of the crushed analyzed subject S through a chemical analysis method. The calorie calculation device 1 uses near-infrared light to calculate the calories of the analyzed subject S. Thus, the calorie calculation device 1 does not need to crush the analyzed subject S and is able to calculate calories in a non-destructive manner.

Further, the chemical analysis technique of the prior art needs to use a reagent and a centrifuge or the like. The calorie calculation device 1 analyzes the calories of the analyzed subject S without using a reagent and a centrifuge or the like. Thus, the calorie calculation device 1 is able to calculate calories with a simple configuration.

(3) Protein does not have a clear absorption spectrum in the band of 700 to 1100 nm. Thus, the measurement accuracy of protein is low. The calorie calculation device 1 calculates the calories of the analyzed subject S without calculating the content ratio of protein in the analyzed subject S. Thus, the calorie calculation device 1 is able to calculate calories without using protein, the measurement accuracy of which is relatively low.

(4) The calorie calculation device 1 calculates calories based on the spectrum data for the band of 700 to 1100 nm. Near-infrared light in the band of 700 to 1100 nm enters the analyzed subject S more easily than near-infrared light of 1100 nm or higher. Thus, compared to when using near-infrared light of 1100 or higher, the calorie calculation device 1 is able to calculate calories reflecting the inner part of the analyzed subject S.

(5) The calorie calculation device 1 includes the case 11 that shields its interior from light. This can reduce the influence of near-infrared light from the outside. Thus, decreases are limited in the calorie calculation accuracy of the calorie calculation device 1.

Second Embodiment

A second embodiment of the calorie calculation device 1 differs from the first embodiment of the calorie calculation device 1 in that the calorie calculation device 1 measures fat in the analyzed subject S in addition to water. Otherwise the second embodiment is the same as the first embodiment.

The calorie calculation method will now be described.

The control unit 70 spectrally measures the near-infrared light based on a signal from the light receiver 22 and generates spectrum data. The control unit 70 calculates the water ratio of the analyzed subject S based on the spectrum data and the position of the analyzed subject S. The control unit 70 calculates the weight of water (hereinafter referred to as "the water amount W") based on the calculated water ratio and the weight of the analyzed subject S (hereinafter referred to as "the total weight X"). Further, the control unit 70 calculates the fat ratio of the analyzed subject S based on the spectrum data. The control unit 70 calculates the weight of fat (hereinafter referred to as the "fat weight F") based on the calculated fat ratio and the total weight X of the analyzed subject S.

Fat has a calorie coefficient of "9". Protein has a calorie coefficient of "4". Carbohydrate has a calorie coefficient of "4". Fat has a higher calorie coefficient than protein and carbohydrate. Protein and carbohydrate have the same calorie coefficient. Thus, as long as the sum of the amount of protein and the amount of carbohydrate can be estimated, calories derived from protein and carbohydrate can be calculated without measuring each of the amount of protein and the amount of carbohydrate. Protein, carbohydrate, fat, and water take up most of the weight of a typical food. Thus, a value obtained by subtracting the water amount W from the total weight X is substantially equal to the amount of protein and the amount of carbohydrate. Thus, the control unit 70 calculates the calories with equation (1), which is shown below, based on the water amount W and the fat amount F.

$$C=(X-W-F)\times 4+F\times 9 \quad (1)$$

Here, "C" represents the calories of the analyzed subject S.

In addition to advantages (2) to (5) of the first embodiment, the calorie calculation device 1 has the advantages described below in the second embodiment.

(6) The calorie calculation device 1 calculates the calories of the analyzed subject S based on the water amount W and the fat amount F in the analyzed subject S. This allows calories to be calculated with a simpler configuration than a configuration in which the protein, carbohydrate, fat, and water in the analyzed subject S are all measured to calculate the calories.

(7) Fat has the highest calorie coefficient among protein, carbohydrate, and fat. The calorie calculation device 1 performs a measurement on fat in addition to water. Thus, the calorie calculation accuracy is higher than a configuration in which calories are calculated using only water.

Other Embodiments

The calorie calculation device includes embodiments other than the first embodiment and the second embodiment. Modifications of the first embodiment and the second embodiment will now be described as other embodiments of the calorie calculation device. The modifications described below may also be combined with one another.

The calorie calculation device 1 of the first embodiment uses the water ratio to calculate the calories of the analyzed subject S. However, the calorie calculation device 1 is not limited to such a configuration. For example, among the water ratio, the protein ratio, the carbohydrate ratio, and the fat ratio of the analyzed subject S, a modification of the calorie calculation device 1 calculates the calories of the analyzed subject S using one, two, or three of these parameters.

When using one parameter to calculate the calories of the analyzed subject S, the control unit 70 saves, in advance, an equation that indicates the relationship between the parameter and the weight calories. The control unit 70 calculates the calories of the analyzed subject S using the equation that indicates the relationship of the parameter and the weight calorie.

The control unit 70 of the first embodiment calculates the calories of the analyzed subject S based on the water ratio. However, the control unit 70 is not limited to such a configuration. For example, the control unit 70 of a modification calculates a water-excluded amount in which the water amount is excluded from the weight of the analyzed subject S. The control unit calculates the calories of the analyzed subject S from the relationship of the calculated water-excluded amount and the weight calorie, which is set in advance through experiments or the like.

The control unit 70 of the second embodiment uses the water amount W and the fat amount F to calculate the calories. However, the calorie calculation device 1 is not limited to such a configuration. For example, the control unit 70 of a modification uses food type information, in addition to the water amount W and the fat amount F, to calculate calories. The food type information includes the names of foods, for example, salad, rice, bread, and hamburger.

The control unit 70 stores information for a number of food types. A person performing a measurement uses the operation unit 50 to select the food type corresponding to the type of the analyzed subject S. Based on the selected food type information, the control unit 70 calculates the calories. The calories are calculated using, for example, equation (2), which is shown below. In the modification, the calorie calculation device 1 has higher calorie calculation accuracy than a configuration that calculates the calories by only measuring components. Here, "T" indicates a correction value set for each food type. In the modification, the operation unit 50 corresponds to a "food type information input unit". This modification is referred to as modification X.

$$C=(X-W-F-T)\times4+F\times9 \quad (2)$$

Modification X may be further modified as described below. The calorie calculation device 1 of this modification further includes a camera. The control unit 70 processes images captured by the camera to detect the food type information of the analyzed subject S. For example, when a green color occupies a predetermined area or greater of an image, the control unit 70 selects salad from the information of different types of foods stored in advance. In this modification, the control unit 70 corresponds to a "food type information detector".

The calorie calculation device 1 of the second embodiment calculates the calories using a value obtained by subtracting the water amount W and the fat amount F from the total weight X in equation (1) as the sum of the weight of protein and the weight of carbohydrate. However, the calorie calculation device 1 is not limited to such a configuration. For example, the calorie calculation device 1 of a modification calculates the calories using a value obtained by subtracting a small value of salts or the like, the water amount W, and the fat amount F from the total weight X as the sum of the weight of protein and the weight of carbohydrate. Salts have a calorie coefficient of "0". Thus, the use of a value obtained by subtracting the weight of salts from the total weight X allows for improvement of the calorie calculation accuracy. The weight of salts may be calculated with the measurement unit 20 or be stored in advance in the control unit 70 as the average amount of salt for foods.

The weight detector 30 in each embodiment measures the weight of the analyzed subject S with the piezoelectric element 31. However, the weight detector 30 is not limited to such a configuration. For example, the weight detector 30 of a modification may be configured by a camera. The weight detector 30 provides the control unit 70 with detected images. The control unit 70 analyzes the input images to calculate the volume of the analyzed subject S. The control unit 70 calculates the weight of the analyzed subject S based on the volume of the analyzed subject S.

The calorie calculation device 1 of each embodiment includes the weight detector 30 that measures the weight of the analyzed subject S. However, the calorie calculation device 1 is not limited to such a configuration. For example, the calorie calculation device 1 of a modification includes a volume detector instead of the weight detector 30 to measure the volume of the analyzed subject S. For example, the position sensor 40 may be used as the volume detector. When using the position sensor 40 as the volume detector, the calorie calculation device 1 stores, in advance, the distance from the sample tray 12 to the position sensor 42 in the control unit 70. The control unit 70 estimates the thickness of the analyzed subject S based on the distance from the sample tray 12 to the position sensor 42 and the detection value of the position sensor 42. The control unit 70 also estimates the size of the analyzed subject S in the lateral direction based on the detection value of the position sensor 42. The control unit 70 calculates the volume of the analyzed subject S based on the estimated value of the thickness of the analyzed subject S and the estimated value of the size of the analyzed subject S in the lateral direction. The control unit 70 estimates the weight of the analyzed subject S from the volume of the analyzed subject S. In this case, the calorie calculation device 1 stores, in the control unit 70, the average weight per volume obtained from a number of types of food. The control unit 70 multiplies the average weight value by the weight to estimate the weight of the analyzed subject S. This modification is referred to as modification Y.

The calorie calculation device of modification Y estimates the weight of the analyzed subject S from the volume of the analyzed subject and calculates the calories based on the estimated weight and the water ratio. However, the configuration of the calorie calculation device 1 in modification Y may be further modified so that the calorie calculation device 1 calculates the calories based on the volume of the analyzed subject S and the water ratio. In this case, the calorie calculation device 1 uses an equation indicating the relationship between the water ratio, which is obtained using multiple types of foods having different volumes, and the calories per volume (hereinafter referred to as "the size calories"). The control unit 70 uses the equation indicating the relationship between the water ratio and the size calories to calculate the size calories of the analyzed subject S. The control unit 70 calculates the calories of the analyzed subject S based on the size calories of the analyzed subject S and the volume of the analyzed subject S.

The calorie calculation device 1 of each embodiment corrects the water ratio based on the position of the analyzed subject S measured by the two position sensors 41 and 42. However, the calorie calculation device 1 is not limited to such a configuration. For example, Fig. 4 shows a modification of the calorie calculation device 1 including a first movement mechanism 81 and a second movement mechanism 82. The first movement mechanism 81 moves the sample tray 12 in upper and lower directions. The second movement mechanism 82 moves the sample tray 12 in right and left directions. The control unit uses the two movement mechanisms 81 and 82 to change the position of the sample tray 12. The control unit 70 changes the position of the analyzed subject S based on the detection value of the position sensor 40. In this case, a fixed distance may always be maintained from the analyzed subject S to the light emitter 21 and the light receiver 22. Thus, the correction of the water ratio based on the position of the analyzed subject measured by the position sensors 41 and 42 may be omitted. The two movement mechanisms 81 and 82 may be manually driven.

The calorie calculation device 1 of each embodiment includes the position sensor 40. However, the calorie calculation device 1 is not limited to such a configuration. For example, a modification of the calorie calculation device 1 does not include the position sensor 40. In this case, the control unit 70 does not perform calorie correction with the position of the analyzed subject S.

The calorie calculation device 1 of each embodiment shows the calories of the analyzed subject S on the display 60. However, the calorie calculation device 1 is not limited to such a configuration. For example, a modification of the calorie calculation device 1 includes a port that provides an external medium with a calorie calculation result. Examples of the port include a USB and a wireless communication port.

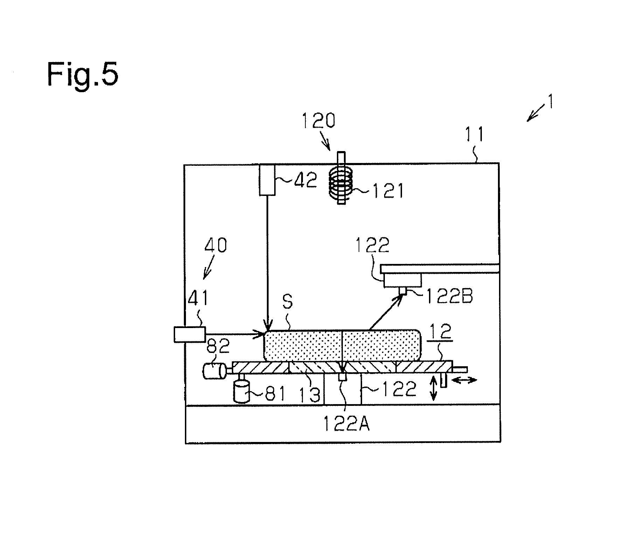

The calorie calculation device 1 of each embodiment calculates the calories using the spectrum data for near-infrared light from 700 to 1100 nm. However, the calorie calculation device 1 is not limited to such a configuration. For example, the calorie calculation device 1 of a modification calculates the calories using spectrum data for near-infrared light of 1100 nm or higher. For example, the calorie calculation device 1 of a modification shown in Fig. 5 includes a measurement unit 120 that uses nuclear magnetic resonance (NMR). The measurement unit 120 includes a magnetic field generator 121 and an electromagnetic wave detector 122. The electromagnetic wave detector 122 includes a first detector 122A and a second detector 122B. The first detector 122A detects electromagnetic waves from the lower side of the analyzed subject S. The second detector 122B detects electromagnetic waves from the upper side of the analyzed subject S. Based on signals from the detectors 122A and 122B, the control unit 70 generates NMR spectrum data. The control unit 70 measures the water in the analyzed subject S based on the spectrum data that corresponds to the chemical structure of water. For example, a coil and a magnet may be used as the magnetic field generator 121. Any measurement unit 20 may be employed as long as the measurement unit 20 is capable of performing a measurement on the water in the analyzed subject S.

The calorie calculation device 1 of each embodiment uses near-infrared light to calculate the calories of the analyzed subject S in a non-destructive manner. However, the calorie calculation device 1 is not limited to such a configuration. For example, the calorie calculation device 1 of a modification crushes the analyzed subject S in a chemical analysis to calculate the calories of the analyzed subject S. More specifically, the control unit 70 measures the calories of the analyzed subject S based on the water measured through the chemical analysis and the weight of the analyzed subject S. In this case, the calorie calculation device 1 may calculate the calories of the analyzed subject S with only water.

The calorie calculation device 1 of each embodiment may be modified by adding the following configuration. The calorie calculation device 1 in the modification uses a reference sample to calculate the calories. More specifically, a user prepares a reference sample, the spectrum data of which changes in the same manner as the analysis subject S depending on the environment, such as the temperature and the humidity. The user measures the calories of the reference sample before measuring the analyzed subject S. For example, when the analyzed subject S is a liquid, water is used as the reference sample. The control unit 70 compares the calculated calories of the reference sample with pre-stored reference calories of the reference sample to determine a correction coefficient. When performing calorie calculation of the analyzed subject S, the control unit 70 uses the correction coefficient determined by the reference sample to correct the calculated calories. This limits decreases in the calorie measurement accuracy that would result from the environment, such as the temperature and the humidity. This modification is referred to as modification Z.

The calorie calculation device 1 of modification Z uses the calories of the reference sample to determine the correction coefficient. Instead, the calories of the reference sample may be used to change the position of the light emitter and the light emitting intensity. More specifically, the position and light emitting intensity of the light emitter are changed so that the calories of the reference sample matches the pre-stored reference calories of the reference sample. This limits decreases in the calorie measurement accuracy that would result from the environment, such as the temperature and the humidity.

The calorie calculation device 1 of each embodiment includes the display 60, which has a liquid crystal screen. However, the calorie calculation device 1 is not limited to such a configuration. For example, the calorie calculation device 1 of a modification includes a display 60 having LEDs. The display 60 may be configured in any manner as long as the calculated calories can be shown.

The following configuration may be added to the calorie calculation device of each embodiment. The relationship of the weight of the analyzed subject S and the calories may be shown on the display 60. In this configuration, when further increasing the weight of the analyzed subject S, the user can easily recognize the final calories. Further, by comparing the target calorie intake amount with the shown calorie per weight of the analyzed subject S, the user may easily compare the target calorie intake amount and the intake amount of the analyzed subject S.

The calorie calculation device 1 of each embodiment includes the sample tray 12. However, the calorie calculation device 1 is not limited to such a configuration. For example, the calorie calculation device 1 of a modification does not include the sample tray 12. In this case, the analyzed subject S may be placed in a silica glass container.

In the calorie calculation device 1 of each embodiment, the position sensor 40 is coupled to the interior of the case 11. However, the calorie calculation device 1 is not limited to such a configuration. For example, the calorie calculation device 1 of a modification couples the position sensor 40 to at least one of the light emitter 21 and the light receiver 22. The modification is referred to as modification V.

In the calorie calculation device 1 of modification V, the measurement value of the position sensor 40 corresponds to the distance between the analysis subject S and the measurement unit 20. Thus, the calorie calculation device 1 may further include a movement mechanism for the light emitter 21 and the light receiver 22 so that a constant distance can be maintained between the distance from the analyzed subject S to the light emitter 21 and the light receiver 22 by using the measurement value of the position sensor 40.

The measurement unit 20 of each embodiment includes two light receivers 22. However, the measurement unit 20 is not limited to such a configuration. For example, the measurement unit 20 of a modification does not include one of the light receivers 22. The measurement unit 20 of a further modification includes three or more light receivers 22. In this case, the light reception element may be configured to receive the light transmitted through the analyzed subject S or the diffused reflection light from the analyzed subject S. The position of the light receiver 22 may be changed in accordance with purpose, the shape of the main body 10, or the like.

Further, in a configuration including a plurality of light reception elements, a light reception element that is mainly used for measurements may be set and the other light reception elements may be arranged nearby. Further, the detection values of the other light reception elements may be used to correct the detection value of the main light reception element.

The measurement unit 20 of each embodiment includes a single light receiver 22 that includes a light reception element 22B to receive diffused reflection light. However, the measurement unit 20 is not limited to such a configuration. For example, the measurement unit 20 of a modification includes light receivers 22, which receive diffused reflection light, and an optical fiber. The optical fiber has an end that is branched. Each light receiver 22 is connected to the branched end. The other end of the optical fiber is connected to a light reception element 22B. The diffused reflection light is collected by the light reception element 22B through the light receivers 22 and the optical fiber. The measurement unit 20 collects the diffused reflection light that reaches a plurality of locations. This allows the intensity of the light that reaches the light reception element 22B to be increased.

The case 11 of each embodiment includes a door. However, the case 11 is not limited to such a configuration. For example, the case 11 of a modification does not include the door. In this case, the calorie calculation device 1 may measure the absorption when the analyzed subject S is not arranged in the case 11 and use this absorption as a reference value for the absorption obtained when measuring the analyzed subject S. More specifically, the calorie calculation device 1 calculates the absorption based on a value obtained by subtracting a value obtained when the analyzed subject S is not arranged in the case 11 from a value measured when the analyzed subject S is arranged in the case 11.

The calorie calculation device 1 of each embodiment includes the case 11. However, the calorie calculation device 1 is not limited to such a configuration. For example, the calorie calculation device 1 of a modification does not include the case 11.

**Claims**

1.   A calorie calculation device comprising:

   a measurement unit that performs a measurement on, at most, three components among water, protein, carbohydrate, and fat that are included in an analyzed subject;
   either a weight detector that measures weight of the analyzed subject or a volume detector that measures volume of the analyzed subject; and
   a calorie calculator that calculates calories of the analyzed subject using a measurement result of the measurement unit and either a measurement result of the weight detector or a measurement result of the volume detector.

2.   The calorie calculation device according to claim 1, wherein the measurement unit performs a measurement on at least water among water, protein, carbohydrate, and fat that are included in the analyzed subject.

3.   The calorie calculation device according to claim 1 or 2, wherein the measurement unit performs a measurement on one or two of protein, carbohydrate, and fat that are included in the analyzed subject.

4.   The calorie calculation device according to any one of claims 1 to 3, wherein the measurement unit performs a measurement on water that is included in the analyzed subject and fat that is included in the analyzed subject.

5.   The calorie calculation device according to any one of claims 1 to 4, wherein the measurement unit measures the analyzed subject based on spectrum data obtained with near-infrared light.

6.   The calorie calculation device according to claim 5, wherein the measurement unit performs a measurement on the analyzed subject based on spectrum data obtained with near-infrared light from 700 to 1100 nm.

7.   The calorie calculation device according to any one of claims 1 to 6, comprising either a food type information input unit that receives food type information of the analyzed subject or a food type information detector that detects food type information of the analyzed subject,
wherein the calorie calculator uses the food type information of the analyzed subject to correct the calories of the analyzed subject.

8.   The calorie calculation device according to any one of claims 1 to 7, wherein
the measurement unit measures a ratio of water included in an analyzed subject, and
the calorie calculator uses the ratio of water included in the analyzed subject and either a measurement result of the weight detector or a measurement result of the volume detector to calculate the calories of the analyzed subject.

9.   The calorie calculation device according to any one of claims 1 to 7, wherein
the measurement unit measures a ratio of water included in an analyzed subject and measures a ratio of fat included in the analyzed subject, and
the calorie calculator uses the ratio of water included in the analyzed subject, the ratio of fat included in the analyzed subject, and either a measurement result of the weight detector or a measurement result of the volume detector to calculate the calories of the analyzed subject.

# Fig.1

# Fig.2

## Fig.3

## Fig.4

Fig.5

**INTERNATIONAL SEARCH REPORT**

International application No.

PCT/JP2013/006955

| A. | CLASSIFICATION OF SUBJECT MATTER |
| --- | --- |

*G01N21/359*(2014.01)i, *G01N21/3554*(2014.01)i

According to International Patent Classification (IPC) or to both national classification and IPC

| B. | FIELDS SEARCHED |
| --- | --- |

Minimum documentation searched (classification system followed by classification symbols)
G01N21/00-21/958

Documentation searched other than minimum documentation to the extent that such documents are included in the fields searched
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X<br>Y | JP 2009-098015 A  (Joy World Pacific Co., Ltd.),<br>07 May 2009 (07.05.2009),<br>entire text; all drawings<br>(Family: none) | 1-7<br>8,9 |
| Y | JP 2011-064518 A  (Panasonic Corp.),<br>31 March 2011 (31.03.2011),<br>entire text; all drawings<br>(Family: none) | 1-9 |
| A | JP 2006-105655 A  (Nippon Telegraph and Telephone Corp.),<br>20 April 2006 (20.04.2006),<br>entire text; all drawings<br>(Family: none) | 1-9 |

☐ Further documents are listed in the continuation of Box C. ☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 18 February, 2014 (18.02.14) | 04 March, 2014 (04.03.14) |

| Name and mailing address of the ISA/<br>    Japanese Patent Office | Authorized officer |
| --- | --- |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002122538 A